(19) 

Europäisches Patentamt
European Patent Office
Office européen des brevets

(11) **EP 3 542 844 B1**

(12) **EUROPEAN PATENT SPECIFICATION**

(45) Date of publication and mention
of the grant of the patent:
**15.04.2026 Bulletin 2026/16**

(21) Application number: **18162855.3**

(22) Date of filing: **20.03.2018**

(51) International Patent Classification (IPC):
**A61M 5/44** (2006.01)   **G01J 5/00** (2022.01)
**G05D 23/27** (2006.01)   **A61M 1/36** (2006.01)

(52) Cooperative Patent Classification (CPC):
**A61M 5/44; G01J 5/0037; G05D 23/1927;**
A61M 2205/3334; A61M 2205/368; A61M 2205/502

(54) **PHYSIOLOGICAL FLUID TEMPERATURE CONTROL APPARATUS**

TEMPERATURREGELUNGSVORRICHTUNG FÜR PHYSIOLOGISCHE FLUIDE

APPAREIL DE RÉGULATION DE TEMPÉRATURE DE FLUIDE PHYSIOLOGIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**25.09.2019 Bulletin 2019/39**

(73) Proprietor: **The Surgical Company International
B.V.
1105 AG Amsterdam (NL)**

(72) Inventor: **VAN KATWIJK, Tim
3821 AH Amersfoort (NL)**

(74) Representative: **Hindles Limited
Clarence House
131-135 George Street
Edinburgh, EH2 4JS (GB)**

(56) References cited:
**EP-B1- 1 313 521       US-A- 5 180 896
US-A1- 2002 011 482    US-A1- 2009 299 271
US-A1- 2011 034 866    US-A1- 2011 315 611
US-A1- 2014 303 608    US-A1- 2016 082 200**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

Field of the invention

The invention relates to the field of physiological fluid temperature control.

Background to the invention

Before physiological fluids are intravenously introduced into the blood stream, it is common to regulate the temperature of the fluids to around body temperature. Devices accomplishing this warming through the use of incandescent bulbs are known (for example, as disclosed in WO0215967 A1) however, in such devices, both the bulbs and the fluids are heated, resulting in low energy efficiency. Furthermore, because both the bulbs and fluid are heated, both emit infrared radiation and, as a result, the temperature of the fluid cannot be reliably distinguished from the temperature of the bulbs using an infrared temperature sensor. This creates difficulties in quickly and reliably adjusting the temperature of the fluid and especially with fine temperature control of the fluid.

EP 1313521 B1 discloses an apparatus for heating blood or another physiological fluid, comprising a conduit through which the fluid can be conducted, and heating means for warming the fluid flowing through the conduit. US 2011/0034866 A1 discloses a system for heating a medical fluid including a container including a fluid path and a broadband infrared radiation source coupled to a broadband emitter which provides a focal location within a fluid path to heat the medical fluid. US 2014/0303608 A1 discloses a system and method for providing treatment feedback for a thermal treatment device. US 2002/0011482 A1 discloses an infrared heating device for prewarming water. US 2009/0299271 A1 discloses a system and method for heating a fluid for delivery of the fluid in the context of a medical, pharmaceutical, industrial, or mechanical application, wherein a heating device is associated with a cassette.

Other known physiological fluid warmers use hot plates or heat exchangers. These also have disadvantages associated with fast and reliable temperature control due to their long response times, long latency and inefficient heat transfer. Many such designs also have further disadvantages associated with difficulties in maintaining sterile conditions for the fluid and disposable conduits are not typically available for such apparatuses.

Accordingly, the invention seeks to address these disadvantages of known physiological fluid temperature control apparatuses.

Summary of the invention

The invention is related to an apparatus for warming or otherwise regulating the temperature of physiological fluids such as (but not limited to) blood, plasma and/or saline. The invention is defined by the appended claims.

A first aspect of the invention provides a physiological fluid temperature control apparatus comprising at least one electroluminescent radiation source, the electroluminescent radiation source comprising a plurality of LEDs, a plurality of temperature sensors, a controller and;

the demountable flow-through component comprising at least one conduit, the conduit comprising a biocompatible plastics material (optionally biocompatible glass) which is permeable to the wavelengths of radiation emitted by the or each radiation source, and/or;
the apparatus having a body, the body comprising a fixture for demountably retaining a demountable flow-through component comprising a said conduit in use,
wherein the temperature sensors are configured to measure the temperature of the fluid in the conduit, wherein the apparatus is configured to direct radiation from the electroluminescent radiation source into a said conduit to thereby heat physiological fluid flowing through a said conduit from a conduit inlet to a conduit outlet in use and wherein the controller is configured to process the output of the plurality of temperature sensors and to regulate the electroluminescent radiation from the at least one electroluminescent radiation source to thereby control the temperature of physiological fluid in the conduit,

wherein the at least one electroluminescent radiation source emits radiation with a wavelength centred between 300 nm and 500 nm,
and wherein the controller is configured to determine the appropriate power output of each LED individually, taking into account the temperature at each temperature sensor, the rate of fluid flow in the conduit, and different target temperatures at different locations by mathematical modelling.

Because the radiation source comprises at least one electroluminescent radiation source rather than at least one incandescent radiation source, a relatively high proportion of the energy supplied to the radiation source is converted into light rather than into heat (i.e. a relatively high proportion of visible radiation is produced, and a relatively low proportion of infrared radiation is produced). Thus, the energy efficiency of the present invention is superior to that of similar apparatuses using, for example, incandescent bulbs.

Although in some embodiments parts of the radiation source may increase in temperature during use (for example, the semiconductor junction in an LED will increase in temperature during use) appropriate heat sinking of such radiation sources prevents any appreciable increase in surface temperature of such radiation sources across a significant surface area. Because the surface temperature of the radiation source does not increase in use, the radiation source does not produce significant amounts of black body radiation and does not produce significant amounts of infrared radiation. Therefore a further advantage of the use of an electroluminescent radiation source is that the radiation source does not continue to heat the fluid in the conduit when the power supplied to the radiation source is reduced or switched off, resulting in shorter latency periods when adjusting the temperature of the fluid in the conduit.

In some embodiments, the apparatus comprises a body (typically including the controller) having a (said) fixture for demountably retaining a demountable flow-through component in use. In this case, the demountable flow-through component is separable from (and/or re-mountable to) the body and comprises a conduit. The body may comprise the at least one electroluminescent radiation source. However, the demountable flow-through component may comprise the at least one electroluminescent radiation source.

In some embodiments, the apparatus is in the form of a kit of parts, the kit of parts comprising the said body and further comprising a demountable flow-through component, the demountable flow-through component comprising the conduit. In this case the body comprises a fixture for demountably retaining the demountable flow-through component.

In some embodiments the demountable flow-through component comprises at least one temperature sensor.

In embodiments with a body having a fixture for retaining a demountable flow-through component and a demountable flow-through component, the flow-through component may comprise a cooperating fixture which cooperates with the fixture of the body.

The or each conduit comprises a wall that is permeable (e.g. translucent or transparent) to the radiation emitted by the or each electroluminescent radiation source during operation, thus electroluminescent radiation (i.e. electromagnetic radiation generated by electroluminescence) may pass through the or each conduit wall and may be absorbed by the fluid therewithin, thereby heating the fluid. The permeable wall of the or each conduit may transmit at least 50%, or more preferably at least 70%, or more preferably at least 90% of the radiation, produced by the at least one radiation source, that is incident upon it.

In some embodiments the conduit has a circular or elliptical cross section. In some embodiments the conduit does not have a circular or elliptical cross section (for example it may have a rectangular cross section). Typically the conduit has a geometry selected to maximise surface area such that a maximal portion of radiation produced by the radiation source in use may reach the surface of the conduit and be transmitted therethrough. Typically, the conduit has a depth (the direction generally parallel to incident light, e.g. a diameter) of at least 1 mm, or preferably at least 1.5 mm or more preferably at least 2 mm. Typically the conduit has a depth (e.g. a diameter) of less than 1.2 cm, or less than 1.1 cm or preferably less than 1.0 cm.

In some embodiments, the apparatus comprises a radiation-collector, which is part of or in thermal communication with the conduit, configured to absorb radiation from the electroluminescent radiation source that passes through the fluid without being absorbed. The radiation-collector may extend along the full length of the or each conduit. The radiation-collector may be a back plate of the conduit. Preferably the radiation-collector does not extend around the full circumference or outer perimeter of the or each conduit in cross-section. Typically, the radiation-collector is on the side of the conduit opposite where radiation from the at least one radiation source is incident on the conduit, such that the majority or all of the radiation from the at least one radiation source incident on the radiation-collector has passed through the conduit first. Typically, the radiation-collector is on the side of the conduit directly opposite the at least one radiation source. Typically, a radiation permeable region of the conduit is on the side facing the one or more radiation sources. Preferably the back plate is positioned on the side of the or each conduit opposite to the side facing the at least one radiation source. The conduit may be formed at least in part, by the radiation-collector and a translucent or transparent wall portion sealedly connected to the radiation-collector to form a tube.

In some embodiments the radiation-collector comprises coloured biocompatible plastics material (which may for example be coated on the inner or outer wall of, or part of the conduit) configured to absorb radiation produced by the radiation source and/or to emit minimal radiation in the infrared regime. In some embodiments the radiation-collector comprises a coating on the external surface of the conduit. In some embodiments the radiation-collector comprises a (e.g. biocompatible) coating on the internal surface of the conduit. In some embodiments the radiation-collector is integrated into the conduit (e.g. a portion of the conduit may comprise biocompatible plastics material which is pigmented or dyed such that the said portion absorbs more and transmits correspondingly less of the radiation incident upon it than the portions of the conduit that are not pigmented or dyed). In some embodiments the radiation collector may comprise any other suitable biocompatible material or materials (for example, glass).

It may be that the radiation-collector has a higher thermal conductivity than the rest of the conduit. It may be that the radiation-collector has a higher specific heat capacity than the rest of the conduit.

In some embodiments, the radiation-collector increases in temperature due to the absorbed radiation incident upon it. The heat from the radiation-collector can then transfer to the fluid in the or each conduit. In this way the apparatus can be used to heat fluids that do not readily directly absorb the radiation, such as those fluids that are transparent at the wavelengths produced by the radiation source in use. It is also helpful to heat liquid in the conduit by a combination of the absorption of electroluminescent radiation from the at least one electroluminescent light source and by thermal conduction from the radiation-collector, because although the heating (and heat capacity) of the radiation-collector leads to conductive heating which cannot be varied as quickly as radiative heating, the electroluminescent radiation from the at least one electroluminescent light source can be rapidly varied and so the total rate of heating can still be varied with low latency.

At least part of the wall of the or each conduit is at least partially permeable to the wavelengths of radiation emitted by the or each radiation source to enable radiation to enter the interior of the conduit, however there may be at least part of the wall of the or each conduit which is not permeable to the wavelengths of radiation emitted by the or each radiation source.

The or each conduit comprises biocompatible plastics material and may be disposable. The or each conduit may comprise any other suitable biocompatible material or materials (for example glass). Parts of the conduit may be permeable to visible radiation. All of the conduit may be permeable to visible radiation. It may be that some or all of the conduit is transparent to visible radiation. It may be that some or all of the conduit is not transparent to visible radiation.

According to the invention, the at least one electroluminescent radiation source comprises a plurality of LEDs. In further embodiments not according to the invention, the at least one electroluminescent radiation source comprises at least one laser. The at least one electroluminescent radiation source emits radiation with a wavelength centred between 300 nm and 500 nm, or more preferably between 390 nm and 410 nm.

It may be that the wavelength spectrum of radiation produced by one electroluminescent radiation source differs to the wavelength spectrum of the radiation produced by one or each other radiation source. It may be that the wavelength spectrum of radiation produced by one electroluminescent radiation source is substantially the same as that produced by each other radiation source. It may be that power is supplied to each radiation source independently. It may be that each radiation source shares the same common power supply with each other radiation source.

In some embodiments, the or each conduit is arranged around the radiation source or sources, for example helically, however, one skilled in the art will appreciate that other configurations would also be effective, provided that the radiation sources are positioned such that the radiation can pass through the conduit walls and be absorbed by the fluid. For example, the or each conduit may be arranged in a serpentine configuration. Or, for example, the radiation sources could be placed outside of the or each conduit coil.

The physiological fluid temperature control apparatus comprises a plurality of temperature sensors, which may be, for example, arranged in an array, or spaced along the length of the conduit. At least one temperature sensor may be an infrared temperature sensor (e.g. a thermopile, pyroelectric detector, bolometer, quantum detector or other suitable infrared detector). In embodiments wherein at least one temperature sensor is an infrared temperature sensor, the at least one temperature sensor may be configured to have a peak sensitivity at a wavelength which is above 800 nm, or more preferably above 1000 nm, or above 1400 nm in operation. At least one temperature sensor may be configured to be insensitive to radiation with wavelengths that are the same as those wavelengths of radiation produced by the electroluminescent radiation sources in use. This is especially useful where the at least one temperature sensor is an infrared sensor. In embodiments wherein at least one temperature sensor is an infrared temperature sensor, the at least one temperature sensor may be configured to measure the temperature of the fluid in the conduit at a point in the conduit that is permeable to infrared radiation, however the at least one infrared temperature sensor may be configured to measure the temperature of the fluid indirectly (e.g. by measuring the temperature of the radiation-collector or another component in thermal communication with fluid in the conduit).

At least one temperature sensor may be a thermometer. It may be that at least one temperature sensor is not in direct contact with the fluid flowing in the conduit. It may be that at least one temperature sensor is in direct contact with the fluid flowing in the conduit. It may be that at least one temperature sensor is in direct contact with a thermal-conductor which is in direct contact with the fluid flowing in the conduit. It may be that the apparatus further comprises a plurality of photosensors (for example, photodiodes). It may be that at least one temperature sensor is in direct contract with the radiation collector and measures the temperature of the radiation collector.

Typically at least one temperature sensor will record the temperature to an accuracy of ±1°C or better in use, preferably one or more temperature sensor will record the temperature to an accuracy of ±0.5°C or better in use, more preferably at least one temperature sensor will record the temperature to an accuracy of ±0.2°C or better in use. It may be that at least one temperature sensor is configured to record the temperature to a greater degree of accuracy within a predetermined temperature range including 37°C (e.g. between 25°C and 45°C) than outside of that temperature range.

In some embodiments, the physiological fluid temperature control apparatus further comprises at least one flow meter (for example an optical flow meter) configured to measure the rate of flow of fluid through the conduit. It may be that the at least one flow meter is in direct contact with the fluid in the conduit. It may be that the at least one flow meter is not in direct contact with the fluid in the conduit.

Typically, the apparatus will comprise a controller (e.g. an electronic feedback circuit or a processor executing a stored program) configured to adjust the output of radiation from the electroluminescent radiation source in response to a signal from a user interface component and/or the one or more temperature sensors and/or the one or more photosensors and/or the one or more flow meters. In some embodiments the controller may compare the temperature recorded by a or each temperature sensor (e.g. current temperature sensor data) to a predetermined target temperature or temperature range and may increase power supplied to the or each electroluminescent radiation source if the current measured temperature is below the target temperature or temperature range and/or may decrease power supplied to the or each electroluminescent radiation source if the current measured temperature is above the target temperature or temperature range. For example, the target temperature or temperature range is typically within the range 35°C to 40°C, for example within the range 36.5°C to 38.5°C.

The plurality of temperature sensors, together with the controller, provide the advantage of the option of fine temperature control, allowing a user to monitor the temperature of the fluid at a plurality of points across the conduit and adjust the power supplied to the or each electroluminescent radiation source (or the rate of flow within the conduit) accordingly. This may be an automatic process managed by the controller. This process may be manually adjustable by a user using a user interface. For example, the controller or user may control the power supplied to the electroluminescent radiation sources such that the temperature of the fluid never exceeds a given threshold temperature (for example, 37°C) at any point throughout the conduit. It may be that the controller also takes into account one or more of: the temperatures of fluid at the inlet and/or outlet, the temperature of the conduit at one or more locations; measurement of temperature external to the apparatus (to thereby control for fluctuations in external (e.g. ambient) temperature), and/or the temperatures recorded by the plurality of temperature sensors.

It may be that the controller gives greater priority to the data from some temperature sensors than the data from other temperature sensors and adjusts the power to the radiation sources accordingly; for example, it may be that a temperature sensor located closer to the outlet of the conduit may be prioritised over a temperature sensor closer to the inlet. Alternatively, the controller may asses the data from all temperature sensors with equal priority.

Because of the temperature sensors, flow sensors and/or photosensors and the controller, the temperature of the fluid in the conduit can be adjusted very quickly and accurately, either in response to temperature fluctuations or at the initiation of the fluid temperature control process. Because the fluid is heated by absorbing radiation from electroluminescent radiation sources, rather than predominantly through a heat transfer mechanism, (for example, via radiation from a large piece of heated metal or conduction through a heat sink), there is a comparatively low latency period in the adjustment of the fluid temperature.

In some embodiments the apparatus further comprises one more lenses and/or reflectors configured to direct radiation from the electroluminescent radiation source to the fluid, thus reducing radiation loss and hence allowing improved energy efficiency. In some embodiments the internal walls of the body (and/or the demountable flow-through component) have reflective coatings configured to reflect stray radiation back towards the or each conduit.

A second aspect of the invention provides a method of controlling the temperature of a fluid (e.g. a physiological fluid) flowing through a conduit, the method comprising causing physiological fluid to flow-through an apparatus via a conduit which extends from an inlet to an outlet, directing electroluminescent radiation from one or more electroluminescent radiation sources to pass into the conduit and to be absorbed by the fluid therein, and regulating the electroluminescent radiation output of the one or more electroluminescent radiation sources to regulate the temperature of fluid flowing through the conduit. The apparatus is typically an apparatus according to the first aspect of the invention. The at least one electroluminescent radiation source emits radiation with a wavelength centred between 300 nm and 500 nm.

It may be that the temperature which is regulated comprises or consists of the temperature at the outlet of the conduit. However, the temperature may be regulated at a plurality of locations along the conduit.

The method comprises measuring the temperature at one or more locations (e.g. along the conduit) with a plurality of temperature sensors and taking into account the one or more temperature measurements in regulating the electroluminescent radiation output of the one or more radiation sources. Typically the method comprises measuring the temperature of liquid at at least one location in the conduit. The temperature of liquid within the conduit may be measured directly, or indirectly by measuring the temperature of a component in thermal communication with fluid in the conduit.

The method may comprise measuring the rate of flow at one or more locations within the conduit with one or more flow sensors and taking into account the one or more flow rate measurements in regulating the electroluminescent radiation output of the one or more radiation sources.

Description of the Drawings

An example embodiment of the present invention will now be illustrated with reference to the following Figures in which:

Figure 1 is a cut-away schematic diagram of a physiological fluid temperature control apparatus according to the invention;

Figure 2 is a cross section through a conduit in a physiological fluid temperature control apparatus;

Figure 3 is a schematic diagram of a second example arrangement of the apparatus;

Figure 4 is a flow diagram of the temperature control steps carried out by the controller.

Detailed Description of an Example Embodiment

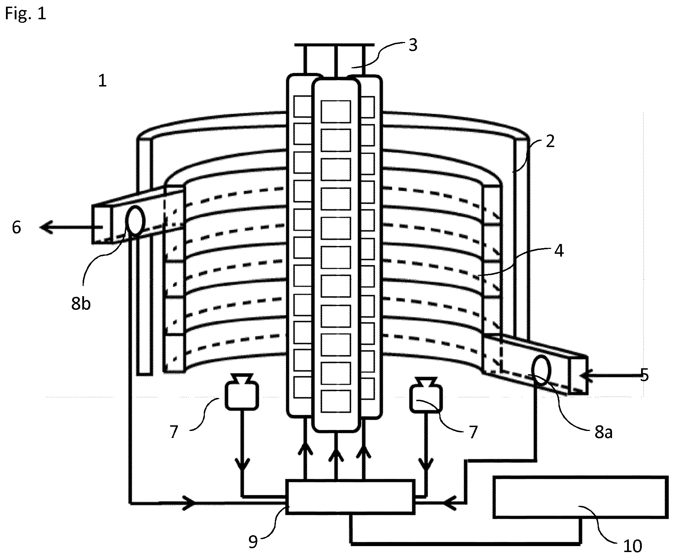

With reference to Figure 1, a physiological fluid temperature control apparatus (1) according to the invention comprises a conduit (4) through which fluid can flow in use, from an inlet (5) to an outlet (6). The conduit is housed within a body (2) and may either be integrally fitted to the body or demountably attached using cooperating fitments in which case the conduit (4) may be disposable. The conduit (4) is a tube of biocompatible plastics material, configured in a helical coil around a plurality of radiation sources, in this example an array of LEDs (3) configured to irradiate the conduit (4). The body (2) acts as a housing for the apparatus and has a reflective coating on its inner wall to reflect any stray radiation back towards the conduit (4).

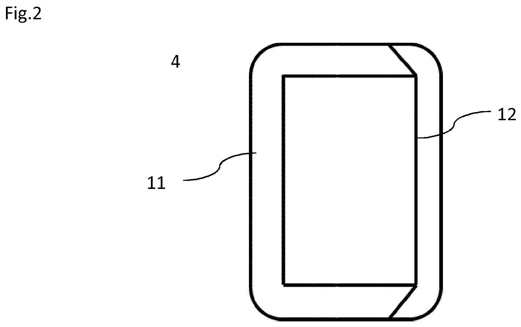

The conduit has a substantially transparent region (11) and a substantially opaque back plate (12), which is sealed to the substantially transparent region and which functions as the radiation collector (Figure 2 shows a cross section through an example conduit). The transparent region (11) comprises transparent biocompatible plastics material and the back plate (12) comprises black biocompatible plastics material. The arrangement is such that, in use, electroluminescent radiation from the LEDs (3) is shone through the transparent region (11) of the conduit (4) and absorbed by the fluid in the conduit (4), with any remaining unabsorbed radiation being absorbed by the back plate (12).

In use, fluid enters the conduit (4) via the inlet (5) and leaves the conduit via the outlet (6). Radiation from the LEDs (3) passes through the conduit (4) and heats the fluid without the LEDs (3) ever being in direct contact with the fluid, thus allowing the heating of the fluid to take place very hygienically. Any radiation produced by the LEDs (3) that is transmitted through the fluid in the conduit (4) without being absorbed by the fluid is absorbed instead by the back plate (12). The temperature of the back plate (12) is thus increased as the back plate (12) is heated by the radiation. The heat from the back plate (12) can then be transferred to the fluid as in more traditional fluid warming apparatuses but with the benefit that the instantaneous rate of heat transfer can still be varied rapidly by varying the LEDs.

The LEDs (3) radiate electroluminescent radiation with a wavelength centred around 400 nm in use. Infrared temperature sensors (7a, 7b) measure the temperature of the fluid in the conduit at multiple positions, spaced along the length of the conduit. For clarity, Figure 1 shows only two temperature sensors (7a, 7b), but more may be in use. In particular, temperature sensors (7a, 7b) monitor the temperature at the inlet (5) and outlet (6) of the conduit (4) - hence temperature data may be collected at various points across the length of the conduit (4) and temperature fluctuations noted. There may be a further temperature sensor to measure the temperature of the back plate. Flow sensors (8a, 8b) measure the rate of flow within the conduit (4). Temperature sensor data (14) is collected by a controller (9) and is compared to a target temperature (11), which can be a discrete value or a range, selected by a user via a user interface (10). The controller (9) automatically compares (15) the current temperature sensor data (14) to the target temperature or range of temperatures (11). If the current temperature indicated by the sensor data (14) is not within the predetermined temperature range (11) the controller adjusts (16) the power supplied (11) to the LEDs (3), either increasing the power (12) if the temperature sensor data (14) indicates that the temperature is below the target or range and decreasing the power (12) if it is above the target or range. According to the invention, the controller determines the appropriate power output of each LED individually taking into account the temperature at each temperature sensor, the rate of fluid flow in the conduit, the absorption properties of the liquid in the conduit, the temperature of the back plate, different target temperatures at different locations etc. by mathematical modelling.

The LEDs are chosen to radiate electroluminescent radiation with a wavelength centred at 400 nm because this wavelength is strongly absorbed by blood. The power supplied to the LEDs is dependent on the rate of fluid flow within the conduit and can be calculated using the following equation:

$$P = (T - T_0)CQ$$

Wherein P is the power supplied to the LEDs, T is the desired temperature of the fluid, $T_0$ is initial temperature of the fluid (i.e. the temperature of the fluid when it enters the conduit), C is the specific heat capacity of the fluid and Q is the rate of flow of the fluid.

Flow sensor (8a, 8b) data is also collected by the controller (9) and the controller (9) increases the power supplied to the LEDs (3) if the flow increases and decreases the power supplied to the LEDs (3) if the flow decreases. The predetermined temperature range and the behaviour of the controller can be adjusted by the user at the user interface (10).

Because LEDs (3) are used to heat the fluid rather than incandescent bulbs, less energy is required. Further, because the temperature of LEDs (3) does not appreciably increase in use, the infrared temperature sensors (7a, 7b) can be used to reliably measure the temperature of the fluid, rather than the temperature of the LEDs (3). This then allows for better temperature control and more immediate adjustment of the power supplied to the LEDs (3) in response to a temperature change in the fluid. Additionally, because the temperature of the LEDs does not appreciably increase in use, temperature adjustments can be carried out more quickly and with a shorter latency period.

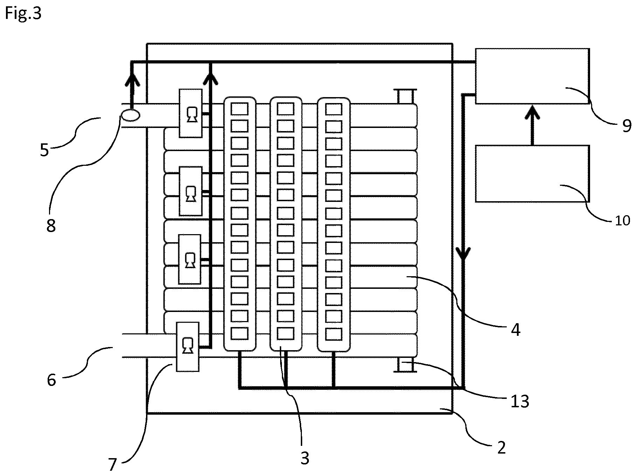

A second example arrangement of the apparatus is shown in Figure 3. Here, the conduit (4) has a serpentine configuration and is demountably held by conduit connectors (13) within the body (2). The LEDs (3) are positioned at one side of the serpentine configuration with the back plate at the opposite side (not shown). An array of temperature sensors (7) monitor the temperature of the fluid within the conduit (4) at several positions including one position close to the inlet (5) and one close to the outlet (6) of the conduit (4). A flow sensor (8) is positioned close to the inlet (5) of the conduit (4). Data from the temperature sensors (7) and the flow sensors (7) is passed to a controller (9) which also receives data from a user interface (10). The controller (9) controls the power supplied to the LEDs (3).

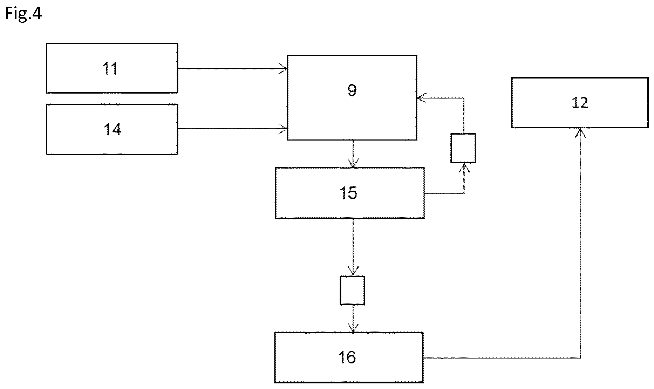

Figure 4 shows an example flow diagram of inputs to and outputs from the controller (9) in an example. A predetermined target temperature range (11) is compared to current temperature sensor data (14) by the controller (9). The controller (9) carries out a first step (15) to determine whether the current temperature sensor data (14) is within the predetermined temperature range (11). If the current temperature sensor data (14) is not within the predetermined temperature range (11), the controller carries out a second step (16) to increase the power (12) supplied to the LEDs (3) if the current temperature sensor data (14) is below the predetermined temperature range (11) or to decrease the power supplied (12) to the LEDs (3) if the current temperature sensor data (14) is above the predetermined temperature range (11).

In some embodiments, the body and channel are integrated. However, in other embodiments, the channel is part of a demountable flow-through unit, which is demountably retained in use by a fixture on the body. In the latter case, the LEDs may be part of the body or part of the demountable flow-through unit. Similarly, some or all of the temperature sensors may be provided in the body; some or all of the temperature sensors may be provided in the demountable-flow through unit. The demountable flow-through unit is typically disposable and may be part of an IV line.

The fixture on the body which retains the flow-through unit may be as simple as a clamp or clip for a tube (e.g. part of an IV line) which functions as the demountable flow-through unit, or may comprise a mounting which slides around, clamps, clips to, holds or otherwise demountable retains the disposable part.

## Claims

1. A physiological fluid temperature control apparatus (1) having a body (2), the body comprising a fixture for demountably retaining a demountable flow-through component in use,

   the apparatus comprising

   at least one electroluminescent radiation source (3), the electroluminescent radiation source comprising a plurality of LEDs a plurality of temperature sensors (7), and a controller (9),

   the demountable flow-through component comprising at least one conduit (4),
   the conduit comprising biocompatible plastics material which is permeable to the wavelengths of radiation emitted by the or each radiation source,
   wherein the temperature sensors are configured to measure the temperature of the fluid in the conduit,
   wherein the apparatus is configured to direct radiation from the electroluminescent radiation source into a said conduit to thereby heat physiological fluid flowing through a said conduit from a conduit inlet (5) to a conduit outlet (6) in use, and
   wherein the controller is configured to process the output of the plurality of temperature sensors and to regulate the electroluminescent radiation from the at least one electroluminescent radiation source to thereby control the temperature of physiological fluid in the conduit,
   wherein the at least one electroluminescent radiation source emits radiation with a wavelength centred between 300 nm and 500 nm,
   and wherein the controller is configured to determine the appropriate power output of each LED individually, taking into account the temperature at each temperature sensor, the rate of fluid flow in the conduit, and different target temperatures at different locations by mathematical modelling.

2. An apparatus (1) according to claim 1, in the form of a kit of parts, the kit of parts comprising
   a body (2) including the said controller (9) and the said fixture,
   the kit of parts further comprising a said demountable flow-through component (4), which optionally comprises the plurality of temperature sensors (7).

3. An apparatus (1) according to claim 1 or claim 2, wherein the apparatus comprises a radiation-collector (12), which is part of or in thermal communication with the conduit (4), configured to absorb radiation from the electroluminescent radiation source (3) that passes through the fluid without being absorbed.

**4.** An apparatus (1) according to claim 3, wherein the radiation-collector (12) is on the side of the conduit (4) opposite where radiation from the at least one radiation source (3) is incident on the conduit, such that the majority or all of the radiation from the at least one radiation source incident on the radiation-collector has passed through the conduit first.

**5.** An apparatus (1) according to claim 3 or claim 4, wherein the radiation-collector (12) comprises a coating on the external or internal surface of the conduit (4).

**6.** An apparatus (1) according to any one of claims 3 to 5, wherein the radiation-collector (12) has a higher thermal conductivity and/or a higher specific heat capacity than the rest of the conduit (4).

**7.** An apparatus (1) according to any one of claims 3 to 6, wherein in use fluid in the conduit (4) is heated by a combination of the absorption of electroluminescent radiation from the at least one electroluminescent light source (3) and by thermal conduction from the radiation-collector (12).

**8.** An apparatus (1) according to any one preceding claim, wherein some or all of the conduit (4) is transparent to visible radiation.

**9.** An apparatus (1) according to any one preceding claim, wherein the plurality of temperature sensors (7) are spaced along the length of the conduit (4).

**10.** An apparatus (1) according to claim 9, wherein the one or more temperature sensors (7) are insensitive to radiation with wavelengths that are the same as those wavelengths of radiation produced by the electroluminescent radiation sources (3) in use.

**11.** An apparatus (1) according to any one preceding claim, further comprising at least one flow meter (8) configured to measure the rate of flow of fluid through the conduit (4).

**12.** An apparatus (1) according to any one of claims 9 to 11, comprising

a plurality of photosensors and
a controller (9) configured to adjust the output of radiation from the electroluminescent radiation source (3) in response to

a signal from a user interface component (10) and/or
the one or more temperature sensors (7) and/or
one or more photosensors and/or
the one or more flow meters (8)

further responsive to a comparison of the temperature recorded by the or each temperature sensor to a predetermined target temperature or range of temperatures.

**13.** A method of controlling the temperature of a fluid flowing through a conduit (4), the method comprising

causing physiological fluid to flow-through an apparatus (1) according to any one preceding claim, via a conduit which extends from an inlet (5) to an outlet (6), and wherein the method further comprises
directing electroluminescent radiation from the one or more electroluminescent radiation sources to pass into the conduit and to be absorbed by the fluid therein,
measuring the temperature at one or more locations with the plurality of temperature sensors and
regulating the electroluminescent radiation output by the one or more electroluminescent radiation sources, taking into account the one or more temperature measurements,

and wherein the at least one electroluminescent radiation source emits radiation with a wavelength centred between 300 nm and 500 nm.

**14.** A method according to claim 13 wherein the apparatus (1) is an apparatus according to any one of claims 1 to 12, the method comprising measuring the temperature at one or more locations with the plurality of temperature sensors (7) and taking into account the one or more temperature measurements when regulating the electroluminescent radiation output by the one or more radiation sources (3) and the rate of flow of fluid at one or more locations within the conduit with one or more flow sensors (8) and taking into account the one or more temperature measurements and one or more flow rate measurements in regulating the electroluminescent radiation output of the one or more radiation sources.

**Patentansprüche**

**1.** Temperaturregelungseinrichtung (1) für physiologische Flüssigkeit, die einen Körper (2) aufweist, wobei der Körper eine Halterung zum abnehmbaren Halten einer abnehmbaren Durchflusskomponente in Verwendung umfasst,

die Einrichtung umfassend
mindestens eine elektrolumineszierende Strahlungsquelle (3), die elektrolumineszierende Strahlungsquelle umfassend: eine Vielzahl von LEDs,

eine Vielzahl von Temperatursensoren (7), und eine Steuerung (9),

wobei die abnehmbare Durchflusskomponente mindestens eine Leitung (4) umfasst,

wobei die Leitung biokompatibles Kunststoffmaterial umfasst, welches für die Wellenlängen der von der oder jeder Strahlungsquelle emittierten Strahlung durchlässig ist,

wobei die Temperatursensoren konfiguriert sind, um die Temperatur der Flüssigkeit in der Leitung zu messen,

wobei die Einrichtung konfiguriert ist, um Strahlung von der elektrolumineszierenden Strahlungsquelle in die Leitung zu lenken, um dadurch physiologische Flüssigkeit, die in Verwendung von einem Leitungseinlass (5) zu einem Leitungsauslass (6) durch die Leitung fließt, zu erwärmen, und

wobei die Steuerung konfiguriert ist, um die Ausgabe der Vielzahl von Temperatursensoren zu verarbeiten, um die elektrolumineszierende Strahlung von der mindestens einen elektrolumineszierenden Strahlungsquelle zu regeln, um dadurch die Temperatur von physiologischer Flüssigkeit in der Leitung zu steuern,

wobei die mindestens eine elektrolumineszierende Strahlungsquelle Strahlung mit einer Wellenlänge emittiert, die mittig zwischen 300 nm und 500 nm liegt,

und wobei die Steuerung konfiguriert ist, um die geeignete Leistungsausgabe jeder LED individuell unter Berücksichtigung der Temperatur an jedem Temperatursensor, der Flüssigkeitsflussrate in der Leitung und verschiedener Zieltemperaturen an verschiedenen Stellen durch mathematische Modellierung zu bestimmen.

2. Einrichtung (1) nach Anspruch 1, in Form eines Teilesatzes, der Teilesatz umfassend:
einen Körper (2), der die Steuerung (9) und die Halterung einschließt, wobei der Teilesatz ferner die abnehmbare Durchflusskomponente (4) umfasst, die optional die Vielzahl von Temperatursensoren (7) umfasst.

3. Einrichtung (1) nach Anspruch 1 oder Anspruch 2, wobei die Einrichtung einen Strahlungskollektor (12) umfasst, der Teil der oder in thermischer Kommunikation mit der Leitung (4) ist, konfiguriert, um Strahlung von der elektrolumineszierenden Strahlungsquelle (3) zu absorbieren, die ohne absorbiert zu werden, durch die Flüssigkeit hindurchgelangt.

4. Einrichtung (1) nach Anspruch 3, wobei der Strahlungskollektor (12) auf der Seite der Leitung (4) gegenüber dem Ort angeordnet ist, wo Strahlung von der mindestens einen Strahlungsquelle (3) auf die Leitung einfällt, so dass das meiste oder alles der auf den Strahlungskollektor einfallenden Strahlung aus der mindestens einen Strahlungsquelle zuerst durch die Leitung hindurchgelangt ist.

5. Einrichtung (1) nach Anspruch 3 oder Anspruch 4, wobei der Strahlungskollektor (12) eine Beschichtung auf der äußeren oder inneren Oberfläche der Leitung (4) umfasst.

6. Einrichtung (1) nach einem der Ansprüche 3 bis 5, wobei der Strahlungskollektor (12) eine höhere thermische Leitfähigkeit und/oder eine höhere spezifische Wärmekapazität als der Rest der Leitung (4) aufweist.

7. Einrichtung (1) nach einem der Ansprüche 3 bis 6, wobei in Verwendung Flüssigkeit in der Leitung (4) durch eine Kombination der Absorption elektrolumineszierender Strahlung aus der mindestens einen elektrolumineszierenden Lichtquelle (3) und durch thermische Leitung aus dem Strahlungskollektor (12) erwärmt wird.

8. Einrichtung (1) nach einem der vorstehenden Ansprüche, wobei ein Teil oder alles der Leitung (4) für sichtbare Strahlung transparent ist.

9. Einrichtung (1) nach einem der vorstehenden Ansprüche, wobei die Vielzahl von Temperatursensoren (7) entlang der Länge der Leitung (4) beabstandet sind.

10. Einrichtung (1) nach Anspruch 9, wobei der eine oder die mehreren Temperatursensoren (7) unempfindlich gegenüber Strahlung mit Wellenlängen sind, die dieselben wie die Wellenlängen der Strahlung sind, die in Verwendung von den elektrolumineszierenden Strahlungsquellen (3) erzeugt wird.

11. Einrichtung (1) nach einem der vorstehenden Ansprüche, ferner umfassend mindestens einen Durchflussmesser (8), der konfiguriert ist, um die Flussrate von Flüssigkeit durch die Leitung (4) zu messen.

12. Einrichtung (1) nach einem der Ansprüche 9 bis 11, umfassend

eine Vielzahl von Photosensoren und
eine Steuerung (9), die konfiguriert ist, um die Ausgabe von Strahlung aus der elektrolumineszierenden Strahlungsquelle (3) als Reaktion auf Folgendes anzupassen:

ein Signal von einer Bedienerschnittstellenkomponente (10) und/oder
den einen oder die mehreren Temperatursensoren (7) und/oder

einen oder mehrere Photosensoren und/oder

den einen oder die mehreren Flussmesser (8)

ferner reagierend auf einen Vergleich der von dem oder jedem Temperatursensor aufgezeichneten Temperatur mit einer vorbestimmten Zieltemperatur oder einem vorbestimmten Bereich von Temperaturen.

13. Verfahren zum Steuern der Temperatur einer durch eine Leitung (4) fließenden Flüssigkeit, das Verfahren umfassend:

Veranlassen, dass eine physiologische Flüssigkeit durch eine Einrichtung (1) nach einem der vorstehenden Ansprüche fließt, über eine Leitung, die sich von einem Einlass (5) zu einem Auslass (6) erstreckt,

und wobei das Verfahren ferner Folgendes umfasst:

Lenken von elektrolumineszierender Strahlung aus der einen oder den mehreren elektrolumineszierenden Strahlungsquellen, um in die Leitung zu gelangen und von der Flüssigkeit darin absorbiert zu werden,

Messen der Temperatur an einem oder mehreren Orten mit der Vielzahl von Temperatursensoren und

Regeln der elektrolumineszierenden Strahlungsausgabe durch die eine oder die mehreren elektrolumineszierenden Strahlungsquellen unter Berücksichtigung der einen oder mehreren Temperaturmessungen,

und wobei die mindestens eine elektrolumineszierende Strahlungsquelle Strahlung mit einer Wellenlänge emittiert, die mittig zwischen 300 nm und 500 nm liegt.

14. Verfahren nach Anspruch 13, wobei die Einrichtung (1) eine Einrichtung nach einem der Ansprüche 1 bis 12 ist,

das Verfahren umfassend Messen der Temperatur an einem oder mehreren Orten mit der Vielzahl von Temperatursensoren (7) und Berücksichtigen der einen oder mehreren Temperaturmessungen beim Regeln der elektrolumineszierenden Strahlungsausgabe durch die eine oder mehreren Strahlungsquellen (3) und der Flussrate von Flüssigkeit an einem oder mehreren Orten innerhalb der Leitung mit einem oder mehreren Flusssensoren (8) und Berücksichtigen der einen oder mehreren Temperaturmessungen und einer oder mehreren Flussratenmessungen beim Regeln der elektrolumineszierenden Strahlungsausgabe der einen oder der mehreren Strahlungsquellen.

**Revendications**

1. Appareil de régulation de température de fluide physiologique (1) ayant un corps (2), le corps comprenant un agencement permettant de retenir de manière démontable un composant à écoulement continu démontable en cours d'utilisation,

l'appareil comprenant
au moins une source de rayonnement électroluminescent (3), la source de rayonnement électroluminescent comprenant une pluralité de DEL
une pluralité de capteurs de température (7), et
un dispositif de commande (9),
le composant d'écoulement continu démontable comprenant au moins un conduit (4),
le conduit comprenant un matériau plastique biocompatible qui est perméable aux longueurs d'onde de rayonnement émis par la ou chaque source de rayonnement,
dans lequel les capteurs de température sont configurés pour mesurer la température du fluide dans le conduit,
dans lequel l'appareil est configuré pour diriger un rayonnement de la source de rayonnement électroluminescent dans un dit conduit afin de chauffer de ce fait un fluide physiologique s'écoulant à travers un dit conduit, d'une entrée de conduit (5) à une sortie de conduit (6) en cours d'utilisation, et
dans lequel le dispositif de commande est configuré pour traiter la sortie de la pluralité de capteurs de température afin de réguler le rayonnement électroluminescent provenant de l'au moins une source de rayonnement électroluminescent pour réguler de ce fait la température du fluide physiologique dans le conduit,
dans lequel l'au moins une source de rayonnement électroluminescent émet un rayonnement avec une longueur d'onde centrée entre 300 nm et 500 nm,
et dans lequel le dispositif de commande est configuré pour déterminer la sortie de puissance appropriée de chaque DEL individuellement, en tenant compte de la température au niveau de chaque capteur de température, du débit d'écoulement de fluide dans le conduit, et de différentes températures cibles au niveau de différents emplacements par modélisation mathématique.

2. Appareil (1) selon la revendication 1, sous la forme d'un kit de pièces, le kit de pièces comprenant un corps (2) comportant ledit dispositif de commande (9) et ledit agencement, le kit de pièces comprenant en outre un dit composant d'écoulement continu démontable (4), qui comprend éventuellement la pluralité de capteurs de température (7).

3. Appareil (1) selon la revendication 1 ou la revendication 2, dans lequel l'appareil comprend un collecteur de rayonnement (12), qui fait partie du conduit (4) ou est en communication thermique avec celui-ci, configuré pour absorber un rayonnement provenant de la source de rayonnement électroluminescent (3) qui traverse le fluide sans être absorbé.

4. Appareil (1) selon la revendication 3, dans lequel le collecteur de rayonnement (12) se trouve du côté du conduit (4) opposé à l'endroit où un rayonnement provenant de l'au moins une source de rayonnement (3) est incident sur le conduit, de telle sorte que la majorité ou la totalité du rayonnement provenant de l'au moins une source de rayonnement incident sur le collecteur de rayonnement a d'abord traversé le conduit.

5. Appareil (1) selon la revendication 3 ou la revendication 4, dans lequel le collecteur de rayonnement (12) comprend un revêtement sur la surface interne externe du conduit (4).

6. Appareil (1) selon l'une quelconque des revendications 3 à 5, dans lequel le collecteur de rayonnement (12) a une conductivité thermique plus élevée et/ou une capacité calorifique spécifique plus élevée que le reste du conduit (4).

7. Appareil (1) selon l'une quelconque des revendications 3 à 6, dans lequel, en cours d'utilisation, le fluide dans le conduit (4) est chauffé par une combinaison de l'absorption de rayonnement électroluminescent provenant de l'au moins une source de lumière électroluminescente (3) et par une conduction thermique provenant du collecteur de rayonnement (12).

8. Appareil (1) selon l'une quelconque revendication précédente, dans lequel une partie ou la totalité du conduit (4) est transparente au rayonnement visible.

9. Appareil (1) selon l'une quelconque revendication précédente, dans lequel la pluralité de capteurs de température (7) sont espacés le long de la longueur du conduit (4).

10. Appareil (1) selon la revendication 9, dans lequel le ou les capteurs de température (7) sont insensibles à un rayonnement dont les longueurs d'onde sont les mêmes que les longueurs d'onde de rayonnement produit par les sources de rayonnement électroluminescent (3) en cours d'utilisation.

11. Appareil (1) selon l'une quelconque revendication précédente, comprenant en outre au moins un débitmètre (8) configuré pour mesurer le débit d'écoulement de fluide à travers le conduit (4).

12. Appareil (1) selon l'une quelconque des revendications 9 à 11, comprenant

une pluralité de photocapteurs et un dispositif de commande (9) configuré pour ajuster la sortie de rayonnement provenant de la source de rayonnement électroluminescent (3) en réponse à un signal provenant d'un composant d'interface utilisateur (10) et/ou le ou les capteurs de température (7) et/ou un ou plusieurs photocapteurs et/ou le ou les débitmètres (8) en réponse en outre à une comparaison de la température enregistrée par le ou chaque capteur de température avec une température cible prédéterminée ou une plage de températures.

13. Procédé permettant de réguler la température d'un fluide s'écoulant à travers un conduit (4), le procédé comprenant

le fait d'amener un fluide physiologique à s'écouler à travers un appareil (1) selon l'une quelconque revendication précédente, par l'intermédiaire d'un conduit qui s'étend depuis une entrée (5) jusqu'à une sortie (6), et dans lequel le procédé comprend en outre le fait de diriger un rayonnement électroluminescent provenant de la ou des sources de rayonnement électroluminescent pour passer dans le conduit et être absorbé par le fluide à l'intérieur de celui-ci, la mesure de la température au niveau d'un ou plusieurs emplacements à l'aide de la pluralité de capteurs de température, et la régulation du rayonnement électroluminescent délivré par la ou les sources de rayonnement électroluminescent, en tenant compte de la ou des mesures de température, et dans lequel l'au moins une source de rayonnement électroluminescent émet un rayonnement avec une longueur d'onde centrée entre 300 nm et 500 nm.

14. Procédé selon la revendication 13, dans lequel l'appareil (1) est un appareil selon l'une quelconque des revendications 1 à 12, le procédé comprenant la mesure de la température au niveau d'un ou plusieurs emplacements à l'aide de la pluralité de capteurs de température (7) et la prise en compte de la ou des mesures de température lors d'une régulation du rayonnement électroluminescent délivré par la ou les sources de rayonnement (3) et du débit d'écoulement de fluide au niveau d'un ou plusieurs emplacements à l'intérieur du conduit à l'aide d'un ou plusieurs capteurs d'écoulement (8) et la prise en compte de la ou des mesures de température et d'une ou plusieurs mesures de débit dans une régulation de la sortie de rayonnement électroluminescent de la ou des sources de rayonnement.

Fig. 1

Fig.2

4

11

12

Fig.3

Fig.4

**EP 3 542 844 B1**

**REFERENCES CITED IN THE DESCRIPTION**

**Patent documents cited in the description**

- WO 0215967 A1
- EP 1313521 B1
- US 20110034866 A1
- US 20140303608 A1
- US 20020011482 A1
- US 20090299271 A1